(19) Europäisches Patentamt
European Patent Office
Office européen des brevets

(11) **EP 3 167 283 B1**

(12) **EUROPÄISCHE PATENTSCHRIFT**

(45) Veröffentlichungstag und Bekanntmachung des Hinweises auf die Patenterteilung:
**31.03.2021 Patentblatt 2021/13**

(21) Anmeldenummer: **15725231.3**

(22) Anmeldetag: **12.05.2015**

(51) Int Cl.:
*G01N 30/46* *(2006.01)*     *C07K 1/16* *(2006.01)*
*G01N 30/88* *(2006.01)*

(86) Internationale Anmeldenummer:
**PCT/EP2015/000978**

(87) Internationale Veröffentlichungsnummer:
**WO 2016/005016 (14.01.2016 Gazette 2016/02)**

(54) **VORRICHTUNG, VERWENDUNG DIESER VORRICHTUNG UND VERFAHREN FÜR DIE STOFFTRENNUNG MIT VERBESSERTER AUSNUTZUNG DER KAPAZITÄT CHROMATOGRAPHISCHER MEDIEN**

DEVICE, USE OF SAID DEVICE, AND METHOD FOR SEPARATING SUBSTANCES WITH AN IMPROVED UTILIZATION OF THE CAPACITY OF CHROMATOGRAPHIC MEDIA

DISPOSITIF, UTILISATION DUDIT DISPOSITIF, ET PROCÉDÉ SERVANT À SÉPARER DES SUBSTANCES EN EXPLOITANT MIEUX LA CAPACITÉ DE MILIEUX CHROMATOGRAPHIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.07.2014 DE 102014010353**

(43) Veröffentlichungstag der Anmeldung:
**17.05.2017 Patentblatt 2017/20**

(73) Patentinhaber: **Sartorius Stedim Biotech GmbH**
**37079 Göttingen (DE)**

(72) Erfinder:
• **VILLAIN, Louis**
**30451 Hannover (DE)**
• **MITTELSTÄT, Jörg**
**37073 Göttingen (DE)**
• **SANTOS DE MATOS FORTUNA, Ana Raquel**
**39108 Magdeburg (DE)**

• **TÖPPNER, Katrin**
**37520 Osterode (DE)**

(74) Vertreter: **Vigand, Philippe**
**Novagraaf International SA**
**Chemin de l'Echo 3**
**1213 Onex - Genève (CH)**

(56) Entgegenhaltungen:
**WO-A1-03/051483     WO-A1-2012/057676**

• **DEANS ET AL: "Use of heart cutting in gas chromatography: A review", JOURNAL OF CHROMATOGRAPHY, ELSEVIER SCIENCE PUBLISHERS B.V, NL, Bd. 203, 9. Januar 1981 (1981-01-09), Seiten 19-28, XP026508537, ISSN: 0021-9673, DOI: 10.1016/S0021-9673(00)80278-2 [gefunden am 1981-01-09]**

EP 3 167 283 B1

**Beschreibung**

Die vorliegende Erfindung betrifft eine Vorrichtung zum Trennen und/oder Isolieren von proteinhaltigen Substanzen in bzw. aus einem Gemisch mit verbesserter Ausnutzung der Kapazität chromatographischer Medien, wobei die Vorrichtung aus einem ersten Chromatographiesystem, einer dem ersten Chromatographiesystem nachgeschalteten zweiten Chromatographiematrix und einem einzigen Sensor zur Detektion der im Fluid enthaltenden Substanzen besteht. Darüber hinaus betrifft die vorliegende Erfindung sowohl die Verwendung dieser Vorrichtung als auch ein Verfahren zum Trennen und/oder Isolieren von proteinhaltigen Substanzen in bzw. aus einem Gemisch. Insbesondere betrifft die vorliegende Erfindung eine Vorrichtung zur Abtrennung mindestens einer Kontaminante von mindestens einer Zielsubstanz in einem flüssigen Medium (Fluid), wobei das flüssige Medium, welches die mindestens eine Kontaminante und die mindestens eine Zielsubstanz enthält, durch die erfindungsgemäße Vorrichtung geleitet wird.

Die Herstellung eines rekombinanten biotechnologischen bzw. biopharmazeutischen Produktes (Protein, Virus, RNA, DNA, Antikörper, Gerinnungsfaktor, Enzym) beinhaltet neben der Expression der Zielsubstanz in Zellkulturen in Fermentern, welche auch als upstream processing (USP) bezeichnet wird, auch die Reinigung der Zielsubstanz von Kontaminanten, die sich zusammen in Lösung befinden (Wirtszellproteine, DNA, RNA, Medienbestandteile, unerwünschte Viren etc.). Letztgenannter Schritt wird auch als downstream processing (DSP) bezeichnet. Der zentrale Schritt im DSP ist die chromatographische Auftrennung der Substanzen, die sich im Zulauf, dem Feedstream befinden. Dies beinhaltet die Bindung von bestimmten Substanzen an eine feste Matrix, die eine Membran, ein partikuläres Gel oder ein Monolith sein kann. Grundsätzlich werden chromatographische Schritte im DSP in zwei Konfigurationen ausgeführt. Diese werden, je nachdem ob die Zielsubstanz bzw. das Zielmolekül an die chromatographische Matrix bindet und anschließend eluiert wird oder in der mobilen Phase verweilt und unerwünschte Kontaminanten an die Matrix binden, als Binden und Eluieren (bind & elute) oder als Durchfluss (flow-through) Anwendungen bezeichnet.

Die Bindungskapazität von chromatographischen Matrizes sowohl für Zielmoleküle oder Kontaminanten ist begrenzt und die Kosten können sehr hoch sein, beispielsweise für die Antikörperaufreinigung mit Affinitätsmatrizes. Dies hat zur Konsequenz, dass sich aus prozessökonomischer Sicht eine günstige Situation dann einstellt, wenn chromatographische Matrizes so klein wie möglich dimensioniert werden und die verfügbare Bindungskapazität möglichst vollständig ausgenutzt wird. Dies wird dadurch limitiert, dass ein Verlust durch Überladen einer Säule im bind & elute Modus bzw. ein Eintrag von Kontaminationen durch ein Überladen der chromatographischen Matrix im flow-through Modus vermieden werden sollte. Der Beladungszustand einer chromatographischen Matrix kann durch einen Sensor, wie beispielsweise einen UV oder IR Sensor, der sich an der Ausgangseite befindet, überwacht werden. Wird die Kapazität einer Matrix überschritten, resultiert dies in einer Änderung der Signalintensität am Ausgang des Mediums. Das Erreichen eines vorher festgelegten Wertes kann als Abbruchkriterium verwendet werden, bei dem die Beladung der Säule gestoppt wird. Ein wichtiger Parameter bei dieser Methode ist das Signal-zu-Rausch Verhältnis gewöhnlicher in Prozessen verwendeter Detektoren, was sich direkt auf die Verluste von Zielmolekül (bind & elute) bzw. auf die Kontamination des Feedstreams mit Nebenkomponenten (flow-through) auswirken kann.

In der Praxis wird deshalb und aufgrund von (i) chargenabhängigen Schwankungen in der Bindungskapazität der chromatographischen Medien, (ii) Fehlern beim maßstäblichen Vergrößern etablierter Prozesse, und (iii) der Notwendigkeit der Berücksichtigung möglicher Grenzfälle, z.B. für Variationen in der Konzentration des Moleküls im Feedstream, aber auch des pH-Wertes und der Leitfähigkeit, zur Sicherheit die theoretisch verfügbare Kapazität einer chromatographischen Matrix nicht vollständig ausgenutzt. Allgemein liegt die Ausnutzung der Kapazität der chromatographischen Matrix schätzungsweise bei nur ca. 60 %. Der Verlust von Zielmolekül bzw. die Kontamination des Prozessstroms kann auf diese Weise zwar vermieden werden, jedoch führt die Überdimensionierung der chromatographischen Matrix zu einer ökonomisch ungünstigen Situation. Eine Optimierung der Ausnutzung der Kapazität der chromatographischen Matrix kann somit ein großes Einsparungspotential bei der Prozesszeit und den Materialkosten, insbesondere bei kostenintensiven chromatographischen Materialien wie Protein A, bieten.

WO 2010/083859 A1 offenbart eine Vorrichtung und ein Verfahren zum Isolieren von Substanzen aus einem Gemisch, wobei die Vorrichtung mindestens eine diffusiv betreibbare und eine konvektiv betreibbare Chromatographiematrix umfasst. Die Verschaltung der diffusiv betreibbaren Matrix (Säule) mit einer kleineren konvektiv betreibbaren Matrix (Membranadsorber) führt in den genannten Beispielen zu einem verbesserten, d.h. steileren Durchbruchverhalten bei der Beladung und einer doppelt so hohen Produktivität (mg/ml x min). Aufgrund der Kombination einer Säule mit einem Membranadsorber kann die Effizienz des Trennprozesses gesteigert und das Durchbruchverhalten des Trennprozesses verbessert werden.

WO 2010/151214 A1 offenbart eine Verschaltung, die den Beladungszustand einer chromatographischen Säule erfasst, indem das Eingangssignal einer Säule über einen ersten Detektor sowie das Ausgangssignal über einen zweiten Detektor erfasst wird. Aus dem Verhältnis des Eingangs- und Ausgangssignals ergeben sich Rückschlüsse auf den Beladungszustand der Säule. Diese Information kann im Kontext eines mehrstufigen chromatographischen Prozesses genutzt werden, um Start und Stop verschiedener Prozessstufen, wie beispielsweise der Beladung festzulegen. Die in WO 2010/151214 A1 offenbarte Vorrichtung erlaubt es, Bindungskapazitäten chromatographischer Säulen zu bestimmen und bedient sich grundsätzlich zweier Sensoren, um den Beladungszustand einer Säule zu ermitteln.

WO 99/34220 A2 offenbart ein Verfahren, mit welchem der Beladungszustand einer chromatographischen Einheit mit einer Lösung, die aus einem Zielmolekül und Kontaminanten besteht, über eine on-line Nachweismethode erfasst werden kann. Dabei wird ein kleiner Teil des Eluats der chromatographischen Einheit aus dem Eluatstrom in eine separate Detektionseinheit abgeführt. Die separate Detektionseinheit, welche z.B. einen chromatographischen Aufbau darstellen kann, ermittelt in weniger als 20 % der gesamten Elutionszeit den Anteil an Kontaminanten und nicht gebundenem Zielmolekül. Die Beladung kann bei Überschreiten einer definierten Konzentration von Zielmolekül im Eluat abgebrochen werden. Bei dieser Methode wird notwendiger Weise ein Teil des Eluats einer chromatographischen Säule in eine separate Detektionseinheit abgeleitet. Eine Rückführung des Anteils des Eluats, welcher für die Analyse verwendet wurde, zurück in den Eluatstrom ist nicht vorgesehen bzw. wird durch die chromatographischen Schritte während der Analyse und die verwendeten Lösungsmittel unmöglich gemacht.

US 7 901 581 B2 offenbart ein System aus mindestens drei Chromatographiematrizes, die durch Ventile miteinander verbunden sind und als "simulated moving bed" Ansatz kontinuierlich betrieben werden können. Das System wird durch eine Kontrolleinheit gesteuert und greift auf UV-Messwerte der Eluate der einzelnen Einheiten zurück. Eine Funktion des Aufbaus ist das Auffangen von nicht gebundenem Zielmolekül aus der Beladungslösung bzw. Waschfraktion einer ersten Säule durch eine nachgeschaltete Säule, wobei mindestens drei getrennt ansteuerbare chromatographische Matrizes benötigt werden. Somit kann verhindert werden, dass nicht gebundenes Zielmolekül aus dem Prozess verloren geht.

EP 1 718 668 B1 offenbart ein Verfahren zur Reinigung von Antikörpern aus einer Kontaminanten enthaltenden Lösung. Dabei wird diese Lösung mit einer chromatographischen Matrix in Kontakt gebracht, auf welcher multimodale Liganden immobilisiert sind. Die multimodalen Liganden umfassen dabei mindestens eine kationentauschende Gruppe und ein aromatisches bzw. heteroaromatisches Ringsystem. Die chromatographische Matrix kann partikulär, ein Monolith oder eine Membran sein. Die antikörperhaltige Lösung, die zum Beladen der Matrix eingesetzt wird, ist dabei ein Eluat aus einem affinitätschromatographischen Schritt wie der Protein A Affinitätschromatographie. Als mögliche Betriebsweisen der multimodalen Chromatographiematrix werden sowohl die flow-through Anwendung, bei der nur Kontaminanten und u.a. auch Protein A adsorbieren, als auch die bind & elute Anwendung, bei der die Antikörper als auch die Kontaminanten adsorbieren und anschließend getrennt voneinander eluiert werden können, genannt. Zwischen den beiden Betriebsweisen kann durch Einstellung des pH-Wertes der Lösung nach dem affinitätschromatographischen Schritt gewechselt werden. Die hier genannte Offenbarung beschreibt den Einsatz einer multimodalen Chromatographiematrix allein oder in einer Zweisäulenanordnung nach einem affinitätschromatographischen Schritt mit dem Ziel einer möglichst wirtschaftlichen Reinigung von Antikörpern. Bei der Zweisäulenanordnung stehen die Vorteile der Kombination zweier unterschiedlicher Matrizes (Affinitätschromatographie und multimodale Kationenaustauschchromatographie) und somit zweier unterschiedlicher Adsorptionsmodi im Vordergrund. Diese erlauben beispielsweise das Binden von ausgewaschenem Protein A oder Kontaminanten, die von Protein A mit dem Antikörper co-eluieren, auf der multimodalen Matrix, während der Antikörper in der mobilen Phase bleibt oder getrennt von den Kontaminanten eluiert werden kann. Die Beladungskapazität der Protein A Säule kann in diesem Verfahren strenggenommen nicht effizienter ausgenutzt werden, da (i) ein Sensor zur Detektion des Durchbruchs nach dem affinitätschromatographischen Schritt fehlt, (ii) der Durchfluss beim Beladen der affinitätschromatographischen Säule nicht auf die multimodale Matrix geleitet wird und (iii) das Eluat des affinitätschromatographischen Schrittes auf die multimodale Matrix geladen wird. Somit sind Einsparungen bei der Kapazität der affinitätschromatographischen Matrix mit diesem Verfahren kaum möglich.

Bei dem Beladen einer chromatographischen Säule mit dem Feedstream, der ein Zielmolekül enthält, wird der Beladungsvorgang oftmals vorzeitig abgebrochen, um ein Überladen der Säule und somit einen Verlust von Zielmolekül zu vermeiden. Die verfügbare Kapazität der Säule wird somit nicht vollständig ausgenutzt. Besonders bei teuren Säulenmaterialien, wie beispielsweise Protein A, ergibt sich so eine ökonomisch unvorteilhafte Situation. Des Weiteren ist die Anzahl der DSP Zyklen pro Batch bzw. Durchlauf in der Regel durch die Größe des verfügbaren Säulenmaterials definiert. Somit können sich als weitere Konsequenz eine längere Gesamtdauer und eine verringerte Effizienz des Prozesses ergeben.

Der vorliegenden Erfindung liegt somit die Aufgabe zugrunde, eine Vorrichtung bereitzustellen, welche die vorstehend genannten Nachteile überwindet und zu einer verbesserten Ausnutzung der Kapazität chromatographischer Medien führt.

Diese Aufgabe wird durch die in den Ansprüchen charakterisierten Ausführungsformen gelöst.

Insbesondere wird eine Vorrichtung zum Trennen und/oder Isolieren von proteinhaltigen Substanzen in bzw. aus einem Gemisch in einem Fluid bereitgestellt, bestehend aus einem ersten Chromatographiesystem, welches eine oder mehrere Chromatographiematrizes umfasst, einem einzigen, dem ersten Chromatographiesystem nachgeschalteten Sensor zur Detektion der im Fluid enthaltenden Substanzen und einer dem Sensor nachgeschalteten zweiten Chromatographiematrix, wobei die zweite Chromatographiematrix dem ersten Chromatographiesystem so nachgeschaltet ist, dass das das Chromatographiesystem verlassende Fluid durch die zweite Chromatographiematrix leitbar ist, wobei die Kapazität der zweiten Chromatographiematrix kleiner der Kapazität des ersten Chromatographiesystems ist und wobei die zweite Chromatographiematrix eine konvektive Chromatographiematrix wie ein Membranadsorber oder ein Monolith ist. Die Chromatographiematrizes eines Chromatographiesystems können darin parallel oder seriell miteinander verbunden sein.

Überraschenderweise wurde festgestellt, dass die Kapazität eines chromatographischen Mediums durch Nachschalten eines Sensors zur Detektion der im Fluid enthaltenden Substanzen und einer kleineren chromatographischen Matrix, die nur einen geringen Teil der Kapazität des ersten Chromatographiesystems, welches beispielsweise auch als Hauptsäule bezeichnet werden kann, aufweist, die Kapazität der Hauptsäule beim Beladen deutlich effizienter ausgenutzt werden kann, ohne dass ein Produktverlust auftritt.

Die erfindungsgemäße Vorrichtung umfasst einen Sensor, der einer zweiten chromatographischen Matrix vorgeschaltet ist, die ihrerseits wiederum einem ersten chromatographischen System nachgeschaltet ist. Die vorliegende Erfindung unterscheidet sich demnach von WO 2010/151214 A1 darin, dass mit Hilfe von nur einem einzigen Sensor der Beladungszustand des ersten chromatographischen Systems (Hauptsäule), d.h. der Durchbruch des Zielmoleküls bzw. der Kontaminante und somit die Überladung der Säule, anhand einer Änderung der Steigung des Durchbruchssignals festgestellt werden kann. Erfindungsgemäß kann folglich auf einen weiteren Sensor, der das Eingangssignal einer Säule über einen vorgeschalteten Detektor erfasst, verzichtet werden. Dabei wird, bei ausreichender Dimensionierung der nach dem Sensor geschalteten zweiten chromatographischen Matrix, eine frühzeitige Detektion des Durchbruchs des Zielmoleküls im Falle eines bind & elute Modus bzw. der Kontaminante im Falle eines flow-through Modus möglich, ohne dass Material in eine separate Analyseneinheit abgeführt werden muss, wie dies in WO 99/34220 A2 zwingend erforderlich ist. Des Weiteren eignet sich die erfindungsgemäße Vorrichtung im Gegensatz zu US 7 901 581 B2 für den Batchbetrieb und kann im Gegensatz zu EP 1 718 668 B1 zu einer Einsparung teurer chromatographischer Matrizes, wie beispielsweise Protein A, führen.

Gemäß der vorliegenden Erfindung unterliegt der Ausdruck "Trennen und/oder Isolieren", wie hierin verwendet, keiner speziellen Einschränkung und betrifft jeden Vorgang, der geeignet ist, eine Substanz aus einem Gemisch zu entfernen. Hierbei bedeutet der Ausdruck "Trennen und/oder Isolieren" beispielsweise, dass eine gewünschte Zielsubstanz aus einem Gemisch, welches in einem Fluid vorliegt, im Sinne einer Reinigung isoliert wird, oder dass eine Verunreinigung entfernt wird, um das Gemisch von dieser Verunreinigung zu befreien. Auch beinhaltet dieser Ausdruck keinerlei Einschränkung bezüglich der zu trennenden Mengen und betrifft sowohl die Trennung analytischer Substanzmengen als auch die präparative Aufreinigung größerer Substanzmengen, die beispielsweise im Rahmen einer Produktion anfallen können.

Darüber hinaus betrifft der Ausdruck "Gemisch" jedes Gemisch, welches mehr als eine Substanz enthält. Gemäß der vorliegenden Erfindung kann ein solches Gemisch eine Komponente in großem Überschuss enthalten, wobei die restlichen Komponenten des Gemisches in kleinen oder kleinsten Mengen vorhanden sind. Der Begriff "Gemisch" beinhaltet jedoch auch solche Gemische, welche mehrere Komponenten enthalten, die in mittlerer Konzentration vorliegen.

Die erfindungsgemäße Vorrichtung zeichnet sich durch die spezielle Anordnung des ersten Chromatographiesystems und der zweiten Chromatographiematrix sowie des zwischengeschalteten Sensors aus. Das erste Chromatographiesystem und/oder die zweite Chromatographiematrix unterliegen keiner besonderen Einschränkung und können sowohl für einen bind & elute Modus als auch für einen flow-through Modus ausgestaltet sein, wobei ein bind & elute Modus bevorzugt ist. Allerdings handelt es sich vorzugsweise bei dem ersten Chromatographiesystem und der zweiten Chromatographiematrix um solche Materialien, welche denselben Auftrennungsmodus beinhalten. Das heißt, für den Fall, dass das erste Chromatographiesystem beispielsweise als bind & elute Modus in Form einer Haupt(trenn)säule ausgestaltet ist, sollte die nachgeschaltete zweite Chromatographiematrix, welche allgemein auch als Trap- oder Fangsäule bezeichnet werden kann, ebenfalls in diesem Modus betrieben werden. In einer anderen erfindungsgemäßen Ausführungsform arbeiten das erste Chromatographiesystem und die zweite Chromatographiematrix nach unterschiedlichen Auftrennungsmodi.

Erfindungsgemäß kann beispielsweise eine Kombination einer diffusiv betreibbaren und einer kleineren konvektiv betreibbaren Chromatographiematrix, d.h. Trennsäule und Membranadsorber, wie in WO 2010/083859 A1 beschrieben, auf die hier vollumfänglich Bezug genommen wird, eingesetzt werden. Insbesondere werden bevorzugt diffusiv betreibbare Chromatographiematrizes mit regulär oder irregulär geformten Partikeln, welche vorzugsweise ionenaustauschende Gruppen aufweisen, als Trennsäule (entsprechend dem ersten Chromatographiesystem) eingesetzt. Besonders bevorzugt umfasst das erste Chromatographiesystem eine Gelchromatographiematrix. Gemäß einer weiteren bevorzugten Ausführungsform der vorliegenden Erfindung umfasst das erste Chromatographiesystem einen Membranadsorber oder Monolithen. Als konvektiv betreibbare Medien gelten in diesem Fall alle Medien, in denen der Stofftransport im Wesentlichen über konvektive und nicht über diffusive Vorgänge bestimmt wird.

Bei der zweiten Chromatographiematrix der erfindungsgemäßen Vorrichtung handelt es sich vorzugsweise um ein flächenförmig ausgestaltetes Gebilde. Dabei wird unter dem Ausdruck "flächenförmig" ein Gebilde verstanden, bei welchem die Ausdehnung in der x- und y-Achse im kartesischen, dreidimensionalen Koordinatensystem die Ausdehnung in der z-Achse um ein Vielfaches übersteigt. Diese flächigen Gebilde können ihrerseits in Form von Flachmodulen, Stapelmodulen, Wickelmodulen oder Zylindern eingesetzt werden. Unter den flächenförmig ausgestalteten Trägermaterialien sind Filter, Membranen, Vliese, Gewebe oder Kombinationen davon besonders bevorzugt. Eine solche Chromatographiematrix kann auch als Bestandteil des ersten Chromatographiesystems eingesetzt werden.

Unter "Trägermaterialien" sollen Materialien verstanden werden, auf deren Oberfläche funktionelle Gruppen gebunden sind, die es erlauben, Substanzen aus flüssigen oder gasförmigen Fluiden zu binden. Die Trägermaterialien als solche sind jedoch in der Regel nicht adsorptiv.

In einer weiteren bevorzugten Ausführungsform ist die zweite Chromatographiematrix monolithisch, d.h. sie ist nicht aus einer Vielzahl von Partikeln aufgebaut, sondern stellt einen einstückigen, zusammenhängenden und porösen Monolithen dar. Kommerziell erhältliche, monolithische Matrizes werden von den Firmen BIA, Ljubljana, Slowenien, (CIM-Disks), und Biorad, Hercules Ca., USA, (UNO Monolith) vertrieben. Eine solche Chromatographiematrix kann auch als Bestandteil des ersten Chromatographiesystems eingesetzt werden.

Eine weitere Ausführungsform der vorliegenden Erfindung betrifft eine wie vorstehend definierte Vorrichtung, wobei die zweite Chromatographiematrix ein Membranadsorber mit mindestens einer Membran ist. Als zweite Chromatographiematrizes sind erfindungsgemäß am meisten bevorzugt Membranadsorber verwendbar, welche mindestens eine mikroporöse Membran umfassen, die optional auf ihrer inneren und äußeren Oberfläche funktionelle Gruppen trägt, welche mit den Substanzen in physikalische und/oder chemische Wechselwirkung treten.

Unter "chemischer Wechselwirkung" soll nachfolgend jedwede kovalente, ionogene und/oder auf elektrostatischen oder hydrophoben (z.B. Van-der-Waals-Wechselwirkung) Interaktionen beruhende Bindungswechselwirkung zwischen den funktionellen Gruppen und den Adsorbenden verstanden werden.

Darüber hinaus werden vorzugsweise poröse, partikuläre Adsorbentien mit regulär oder irregulär geformten Partikeln, welche vorzugsweise ionenaustauschende Gruppen aufweisen, insbesondere Gelchromatographiematrizes als zweite Chromatographiematrix eingesetzt. Solche Chromatographiematrizes können auch als Bestandteil des ersten Chromatographiesystems eingesetzt werden.

Gemäß der vorliegenden Erfindung bedeutet der Ausdruck "nachgeschaltet" lediglich, dass die zweite Chromatographiematrix in Fliessrichtung hinter dem Sensor angeordnet ist, welcher wiederum in Fliessrichtung hinter dem ersten Chromatographiesystem angeordnet ist, und ist darüber hinaus nicht als einschränkend zu verstehen. Dies gilt entsprechend für den Ausdruck "vorgeschaltet". Vorzugsweise ist das das erste Chromatographiesystem verlassende Fluid vollständig durch die zweite Chromatographiematrix leitbar, sodass das gesamte Fluid, welches aus dem ersten Chromatographiesystem austritt und mittels zwischengeschaltenem Sensor analysiert wird, direkt und vollständig durch die zweite Chromatographiematrix geleitet werden kann.

Gemäß einer besonders bevorzugten Ausführungsform der vorliegenden Erfindung liegt die Kombination von Chromatographiesystem, Chromatographiematrix und des Sensors in einer kompakten bzw. einheitlichen Baugruppe vor, sodass eine einfache Handhabung, beispielsweise zur Wartung oder zum Austausch der Vorrichtung, unproblematisch erfolgen kann.

Der erfindungsgemäß verwendete Begriff "Chromatographiematrix" umfasst jedes Material, an dessen Oberfläche mindestens eine Komponente eines Fluids, welches mit dem Material in Kontakt steht, gebunden werden kann. Die Fähigkeit zur Adsorption im Sinne der vorliegenden Erfindung kann dem Material der "Chromatographiematrix" inhärent sein, wie beispielsweise im Falle von Aktivkohle oder Hydroxylapatit, sie kann jedoch auch durch Modifizierung eines Trägermaterials mit einem oder mehreren Liganden erzielt werden, wobei das erste Chromatographiesystem und/oder die zweite Chromatographiematrix mindestens einen Liganden umfasst, der über mindestens eine chemische und/oder physikalische Wechselwirkung mit mindestens einer Substanz in dem Gemisch interagiert. Eine Chromatographiematrix kann Bestandteil des ersten Chromatographiesystems und der zweiten Chromatographiematrix sein.

Beispiele für derart verwendbare Liganden sind Liganden, die über mindestens eine chemische und/oder physikalische Wechselwirkung mit Adsorbenden interagieren, insbesondere Ionenaustauscher, salztolerante Liganden, Chelatbildner, thiophile oder hydrophobe Liganden verschiedener Kettenlängen und Konfigurationen, "Reversed-Phase"-Liganden, reaktive und andere Farbstoffe, anorganische Moleküle und Ionen sowie deren organische und anorganische Verbindungen, Affinitätsliganden, darunter niedermolekulare ungeladene oder geladene organische Moleküle, Aminosäuren und deren Analoga, Coenzyme, Cofaktoren und deren Analoga, Substrate und deren Analoga, endokrine und exokrine Substanzen wie Hormone und hormonähnlich wirkende Effektoren sowie deren Analoga, Enzym-Substrate, -Inhibitoren und deren Analoga, Fettsäuren, Fettsäurederivate, konjugierte Fettsäuren und deren Analoga, Nukleinsäuren wie DNS und deren Analoga und Derivate, RNS und deren Analoga und Derivate, Monomere und deren Analoga und Derivate, Oligo- bis Polymere und deren Analoga und Derivate, hochmolekulare Kohlenhydrate, linear oder verzweigt, unsubstituiert oder substituiert, Glycokonjugate, wie Heparin, Amylose, Zellulose, Chitin, Chitosan, Monomere und Oligomere sowie deren Derivate und Analoga sowie Lignin und dessen Derivate und Analoga.

Weitere Beispiele sind hochmolekulare Liganden wie Proteine und ihre Oligomere, Multimere, Untereinheiten sowie Teile davon, Peptide, Polypeptide, deren Analoga und Derivate, Lectine, Antikörper und Teile davon, Fusionsproteine, Haptene, Enzyme und Untereinheiten sowie Teile davon, Strukturproteine, Rezeptoren und Effektoren sowie Teile davon, Viren und Teile davon, Xenobiotika, Pharmazeutika und pharmazeutische Wirkstoffe, Alkaloide, Antibiotika, Biomimetika und Katalysatoren.

Gemäß einer bevorzugten Ausführungsform der vorliegenden Erfindung umfasst wenigstens das erste Chromatographiesystem und/oder die zweite Chromatographiematrix einen oder mehrere Ligand(en), welcher bzw. welche über mindestens eine chemische und/oder physikalische Wechselwirkung mit Adsorbenden interagiert bzw. interagieren und welcher bzw. welche aus der Gruppe, umfassend Ionenaustauscher, salztolerante Liganden, Chelatbildner, thiophile oder hydrophobe Liganden verschiedener Kettenlängen und Konfigurationen, "Reversed-Phase"-Liganden, reaktive und andere Farbstoffe, anorganische Moleküle und Ionen, deren organische und anorganische Verbindungen, Affinitätsliganden, hochmolekulare Liganden, Enzyme und Untereinheiten sowie Teile davon, Strukturproteine, Rezeptoren und Effektoren sowie Teile davon, Viren und Teile davon, Xenobiotika, Pharmazeutika und pharmazeutische Wirkstoffe, Alkaloide, Antibiotika, Biomimetika und Katalysatoren, ausgewählt ist bzw. sind.

Die Liganden des ersten Chromatographiesystems und der zweiten Chromatographiematrix können gleich oder verschieden zueinander sein und mittels gleicher und/oder verschiedener chemischer und/oder physikalischer Wechselwirkungen mit Adsorbenten interagieren.

Besonders bevorzugte Liganden, die an das erste Chromatographiesystem und/oder die zweite Chromatographiematrix gebunden sind, sind Trimethylamin-, N,N-Diethyl-N-(2-hydroxy- 1-propyl)-ammoniummethyl-, Diethylaminoethyl-, 2,2'-Iminodiethanol-, Carboxymethyl-, Sulfopropyl- und Sulfomethyl-Liganden. Wie vorstehend beschrieben, weisen das erste Chromatographiesystem und die zweite Chromatographiematrix vorzugsweise jeweils mindestens einen Liganden auf, der über dieselbe Art von Bindungswechselwirkung mit den Substanzen in dem Gemisch interagiert. Besonders bevorzugt weisen das erste Chromatographiesystem und die zweite Chromatographiematrix mindestens einen gleichen Liganden auf. Diese Liganden können mit den Adsorbenten über gleiche oder verschiedene chemische oder physikalische Wechselwirkungen interagieren. Weiterhin besonders bevorzugt weisen das erste Chromatographiesystem und die zweite Chromatographiematrix jeweils unterschiedliche Liganden auf. Diese Liganden können mit den Adsorbenten über gleiche und/oder verschiedene chemische und/oder physikalische Wechselwirkungen interagieren.

Erfindungsgemäß weist die zweite Chromatographiematrix eine im Vergleich zum ersten Chromatographiesystem kleinere Kapazität auf. Vorzugsweise weist die zweite Chromatographiematrix eine im Vergleich zum ersten Chromatographiesystem deutlich kleinere Kapazität auf, wobei unter dem Begriff "deutlich kleinere Kapazität" weniger als 50 % der Bindungskapazität des vorgelagerten, ersten Chromatographiesystems verstanden wird. Vorzugsweise beträgt die Kapazität der zweiten Chromatographiematrix weniger als 30 %, mehr bevorzugt weniger als 20 % und besonders bevorzugt weniger als 10 % der Bindungskapazität des vorgelagerten ersten Chromatographiesystems. Andererseits beträgt die Kapazität der zweiten Chromatographiematrix vorzugsweise mindestens 0,005 %, mehr bevorzugt mindestens 0,01 % und besonders bevorzugt mindestens 0,1 % der Bindungskapazität des vorgelagerten ersten Chromatographiesystems, wobei die untere Grenze maßgeblich von der Detektionsempfindlichkeit des nachgeschalteten Detektors abhängig ist, sodass diese zweite Matrix als Trap- oder Fangsäule die durchgebrochenen Zielsubstanzen bzw. Kontaminationen auffangen kann.

Der Begriff "Bindungskapazität" beschreibt die Menge eines Zielmoleküls, das pro Volumeneinheit des chromatographischen Mediums gebunden werden kann (Einheit g/l), und wird abhängig von der zur Bestimmung verwendeten Methode als statische oder dynamische Bindungskapazität bezeichnet. Die dynamische Bindungskapazität eines chromatographischen Mediums ist die Menge an Zielmolekül, welche das Medium unter bestimmten Flussbedingungen binden wird, bevor ein signifikanter Anteil, z.B. 10 % der Konzentration des Moleküls im Feedmedium, durchbricht. Die statische Bindungskapazität eines chromatographischen Mediums ist die Gesamtmenge an Zielmolekül, welche das Medium binden wird und kann unabhängig von der Flussrate, z.B. im Schüttelversuch bestimmt werden. Die statische Bindungskapazität ist somit immer größer als die dynamische Bindungskapazität eines chromatographischen Mediums.

Der Sensor, welcher zwischen dem ersten Chromatographiesystem und der zweiten Chromatographiematrix in der erfindungsgemäßen Vorrichtung angeordnet ist, ist nicht besonders beschränkt, solange dieser zur Detektion der im Fluid enthaltenen Substanzen geeignet ist. Geeignete Sensoren sind dem Fachmann hinlänglich bekannt, wobei beispielsweise die Absorptionsspektroskopie als Detektionsprinzip für den Sensor genannt werden kann. Neben der Absorptionsspektroskopie (insbesondere der UV-, IR-, und Terahertzspektroskopie) ist beispielsweise auch die Verwendung von Refraktometrie, Konduktometrie, Radiometrie, Fluoreszenzspektroskopie insbesondere des Oberflächen-Fluoreszenzquenchings, ATR (Abgeschwächte Total Reflektion) -Infrarotspektroskopie insbesondere der Oberflächenplasmonenresonanzspektroskopie (SPRS), der Manometrie, Potentiometrie, Polarimetrie, Impedanzspektroskopie, NMR-Spektroskopie, Raman-spektroskopie, Turbidimetrie, Nephelometrie, der Ultraschall Laufzeit und die Kombination von mehreren Techniken als Detektionsprinzip für den Sensor möglich. Vorzugsweise umfasst die erfindungsgemäße Vorrichtung einen UV, IR, pH, Druck- oder Leitfähigkeits-Sensor.

Die erfindungsgemäße Vorrichtung umfasst einen Sensor, welcher der zweiten chromatographischen Matrix vorgeschaltet ist, die ihrerseits wiederum dem ersten chromatographischen System nachgeschaltet ist. Erfindungsgemäß ist der Sensor in Bezug auf das Zielmolekül vorzugsweise nicht destruktiv. Die erfindungsgemäße Vorrichtung besteht aus diesen Komponenten. Besonders bevorzugt ist eine Vorrichtung, in welcher ein UV, IR, pH, Druck- oder Leitfähigkeits-Sensor zwischen einem ersten Chromatographiesystem, enthaltend oder bestehend aus einer Gelchromatographiematrix, als Haupttrennsäule und einem nachgeschalteten Membranadsorber als zweite Chromatographiematrix angeordnet ist. Gemäß einer weiteren Ausführungsform der vorliegenden Erfindung kann die Vorrichtung jedoch einen oder mehrere zusätzliche Membranadsorber umfassen, welcher bzw. welche dem Sensor vorgeschaltet ist bzw. sind. Dadurch lässt sich die Detektionsempfindlichkeit für Durchbrüche des Zielmoleküls weiter erhöhen und der Produktverlust kann weiter minimiert werden.

Ein weiterer Aspekt der vorliegenden Erfindung betrifft ein Verfahren zum Trennen und/oder Isolieren von proteinhaltigen Substanzen in bzw. aus einem Gemisch in einem Fluid, umfassend die Schritte des Hindurchleitens des Fluids durch die vorstehend definierte erfindungsgemäße Vorrichtung und des Sammelns des hindurchgeleiteten Fluids.

Gemäß der vorliegenden Erfindung liegt die Flussrate, mit welcher das Fluid durch die vorstehend definierte Vorrichtung hindurchgeleitet wird, in einem Bereich von 0,01 ml/min bis 1000 ml/min, vorzugsweise in einem Bereich von 1 ml/min bis 200 ml/min, besonders bevorzugt in einem Bereich von 2 ml/min bis 100 ml/min und am meisten bevorzugt im Bereich von 2,5 ml/min bis 50 ml/min.

Gemäß der vorliegenden Erfindung liegt bzw. liegen die jeweilige(n) Anfangskonzentration(en) der zu trennenden bzw. zu isolierenden Zielsubstanz(en) in dem Fluid in einem Bereich von 0,001 mg/ml bis 1000 mg/ml, vorzugsweise in einem Bereich von 0,01 mg/ml bis 50 mg/ml, besonders bevorzugt in einem Bereich von 0,1 mg/ml bis 20 mg/ml und am meisten bevorzugt in einem Bereich von 1 mg/ml bis 10 mg/ml, bezogen auf die Einzelsubstanz.

Eine weitere Ausführungsform betrifft ein wie vorstehend definiertes Verfahren, wobei das Gemisch eine Lösung aus einer Synthese, ein biomolekülhaltiges Fluid oder ein biopartikelhaltiges Fluid ist. Unter "biomolekülhaltigem Fluid" soll hierbei ein Fluid verstanden werden, welches Substanzen, ausgewählt aus der Gruppe der Aminosäuren und deren Analoga, Coenzyme, Cofaktoren und deren Analoga, der Endokrine und der exokrinen Substanzen, wie Hormone und hormonähnlich wirkende Effektoren, und deren Analoga, Enzyme und Untereinheiten sowie Teilen davon, Enzym-Substrate, Enzym-Inhibitoren und deren Analoga, Fettsäuren, Fettsäurederivate, konjugierten Fettsäuren und deren Analoga, der Desoxy- und Ribonukleinsäuren und deren Analoga und Derivate, ein bis mehrsträngigen Plasmide, Cosmide und anderer Konstrukte, Kohlenhydrate und Glycokonjugate, Proteine und ihrer Oligomere, Multimere, Untereinheiten sowie Teilen davon, Peptide, Polypeptide, deren Analoga und Derivate, Lektine, Antikörper und Teilen davon, Fusionsproteine, Haptene, Strukturproteine, Rezeptoren und Effektoren sowie Teilen davon, Xenobiotika, Pharmazeutika, Alkaloide, Antibiotika, Biomimetika, Allergene oder Kombinationen davon, enthält.

Unter "biopartikelhaltigem Fluid" soll hierbei ein Fluid verstanden werden, welches Partikel, ausgewählt aus der Gruppe der Viren und Teilen davon, Prionen und Teilen davon, Mikroorganismen, Prokaryonten, Protozoen, Hefen, Pilze, Eukaryonten tierischen und pflanzlichen Ursprungs, Zellwandbestandteile von Prokaryonten, Protozoen, Hefen, Pilze, Eukaryonten tierischen und pflanzlichen Ursprungs und Teilen davon, biologischen Membranen und Teilen davon, subzellulären, biologischen Membranen und Teilen davon oder Kombinationen davon, enthält.

Vorzugsweise werden durch das erfindungsgemäße Verfahren Substanzen erhalten, wobei das Gemisch eine Lösung eines synthetisch oder biologisch hergestellten Produktes, oder ein Naturstoff ist. Darüber hinaus handelt es sich bei dem Gemisch um eine proteinhaltige Lösung, vorzugsweise um eine antikörperhaltige Lösung.

Ein weiterer Aspekt der vorliegenden Erfindung betrifft die Verwendung der erfindungsgemäßen Vorrichtung zum Trennen und/oder Isolieren mindestens eines Proteins in bzw. aus einem Gemisch in einem Fluid. Eine solche Verwendung ist nicht auf einen bestimmten technischen Bereich begrenzt und umfasst alle Anwendungen, in welchen Substanzen aus einem Gemisch getrennt oder isoliert werden müssen. Beispiele solcher Bereiche sind die chemische, pharmazeutische, medizinische oder biologische Forschung oder Produktion.

Erfindungsgemäß kann durch die Verwendung der erfindungsgemäßen Vorrichtung die Kapazität des ersten Chromatographiesystems besser ausgenützt werden. Vorzugsweise wird durch die vorliegende Erfindung eine Ausnutzung der Kapazität des ersten Chromatographiesystems von über 60 %, besonders bevorzugt von über 70 %, ermöglicht.

Die vorliegende Erfindung wird im Folgenden durch Beispiele und anhand der beigefügten Figuren näher beschrieben, ohne jedoch durch diese in irgendeiner Weise eingeschränkt zu sein. Insbesondere sei darauf hingewiesen, dass das UV Signal sowohl nach dem ersten Chromatographiesystem als auch nach der zweiten Chromatographiematrix gemessen wurde, wobei die Messung nach der zweiten Chromatographiematrix nicht notwendigerweise zu dem hier vorgestellten Konzept gehört und nur der Veranschaulichung dient. Die Figuren zeigen:

Figur 1 zeigt den schematischen Aufbau der vorliegenden Erfindung mit Hauptmedium (HM; erstes Chromatographiesystem), Sensor und zweiter Chromatographiematrix (MA).

Figur 2 zeigt die Beladung der HiTrap Protein A Einheit (erstes Chromatographiesystem) mit 140 ml IgG (2,5 mg/ml) und BSA (1 mg/ml) in Puffer A und einer zweiten Chromatographiematrix mit variabler Betthöhe (0 - 4 mm) Sartobind® Protein A (MA). Die gezeigten Durchbruchskurven entsprechen der Messung der Absorption am Ausgang der Säule (Säule) bzw. nach dem Membranadsorber (Säule + MA 0 - 4 mm).

Figur 3 zeigt die Änderung der ersten Ableitung ($\Delta UV/\Delta Volumen$) bzw. des $\Delta UV$ Signals ($\Delta UV = UV_{x+TV} - UV_x$) bei

unterschiedlicher Betthöhe des Membranadsorbers, der dem ersten Chromatographiesystem nachgeschaltet ist. Die Signale wurden aus den UV Absorptionswerten des Sensors, welcher sich unmittelbar nach dem ersten Chromatographiesystem und vor dem Membranadsorber befindet, bestimmt.

Figur 4 zeigt das Verhältnis des Produktverlusts und der Zunahme des Rückdrucks in Abhängigkeit von der Betthöhe des nachgeschalteten Membranadsorbers.

Figur 5 zeigt die Beladung und Elution der HiTrap Protein A Säule (erstes Chromatographiesystem) mit nachgeschalteten Membranadsorbern (zweite Chromatographiematrix) mit 5 cm$^2$ (MA1) und 20 cm$^2$ (MA2) Anströmfläche. Gezeigt sind die Chromatogramme, die mit Hilfe eines Sensors nach der Säule bzw. nach dem Membranadsorber (Säule + MA1 oder MA2) detektiert wurden.

Figur 6 zeigt die UV Absorption (280 nm) während der Beladung des in Tabelle 5 beschriebenen Aufbaus mit Lysozym (5 mg/ml) und Bovinem Serum Albumin (BSA, 2 mg/ml). Durch den zusätzlichen Membranadsorber tritt der Durchbruch des Zielmoleküls erst bei höherem Beladungsvolumen auf (gestrichelte Linie, Detektion nach dem Membranadsorber). Die untere Linie entspricht dem ΔUV-Signal, welches vor dem Membranadsorber gemessen wurde.

**Beispiele**

Materialien und Methoden

Alle Versuche wurden an dem FPLC System ÄktaPrime plus von GE Healthcare durchgeführt. Die UV Absorption wurde bei 280 nm gemessen und Chromatogramme wurden mit Hilfe der Software PrimeView 5.31 (GE Healthcare) ausgewertet. Die Bestimmung der Totvolumina erfolgte durch die Injektion eines nicht bindenden, UVaktiven Tracermoleküls (20 % Aceton), welches dem jeweiligen Equilibrierungspuffer hinzugefügt wurde. Proben bis zu einem Volumen von 150 ml wurden mit Hilfe eines Superloop (GE Healthcare) injiziert. Für Beladungsvolumina über einem Volumen von 150 ml wurden die Proben über eines der Pufferventile der ÄktaPrime aufgetragen. Die Flussrate für die Equilibrierung, die Beladung, das Waschen und die Elution lag bei den Experimenten mit der HiTrap Protein A HP Säule bei 5 ml/min, und bei Experimenten mit Sartobind® Q Nano Kapsulen bei 10 ml/min. Alle Puffersubstanzen waren von Carl Roth (Karlsruhe) oder Merck (Darmstadt). Das Einstellen der pH-Werte der Lösungen erfolgte bei Raumtemperatur.

*Protein A Chromatographie*

Für die Versuche mit der Protein A Chromatographie wurden folgende Puffer verwendet:

A) 1xPBS pH 7,4 (Na$_2$HPO$_4$ (10 mM), KH$_2$PO$_4$ (1,8 mM), NaCl (137 mM), KCl (2,7 mM), 15 mS/cm, und
B) 0,1 M Glycin/HCl, pH = 3,5, 4 mS/cm für die Elution

Zur Beladung wurden unterschiedliche Konzentrationen (0,5-5 mg/ml) von humanem IgG (SeraCare, HS-475) und 1 mg/ml BSA in Puffer A (siehe oben), oder ein vorfiltrierter unverdünnter zellfreier Kulturüberstand einer IgG$_1$-exprimierenden CHO (Chinese Hamster Ovary) Zellline (DG44 Cellca) verwendet. Die detaillierte Beschreibung der Versuchsabläufe befindet sich in den jeweiligen Ausführungsbeispielen.

*Anionentauscherchromatographie*

Für die Versuche mit Anionentauscherchromatographie wurden folgende Puffer verwendet:

C) Tris-HCl pH 7,4 (20 mM) 1,8 mS/cm, und
D) Tris-HCl pH 7,4 (20 mM), NaCl (1 M), 87 mS/cm für die Elution

Zur Herstellung der Beladungslösung wurden Bovines Serum Albumin (BSA) (2 g/l, Kraeber&Co Nr. 4909 2052) und Lysozym (5 g/l, Roth Nr.8259.2) in Puffer C gelöst und über einen Sartolab® Filter (PESU Membran, 0,2 μm Porengröße, Sartorius Stedim Biotech GmbH) filtriert. Zur Untersuchung des Einflusses des Membranadsorbers (siehe Beispiele unten) wurde die Einheit (Sartobind® Q Nano mit und ohne nachgeschalteten Membranadsorber) mit 30 ml Puffer C gespült, anschließend mit 120 ml BSA (2 mg/ml), Lysozym (5 mg/ml) in Puffer C beladen, mit 30 ml Puffer C gewaschen, danach mit 30 ml Puffer D eluiert und schließlich erneut mit 30 ml Puffer C equilibriert.

*Bestimmung des Grads von Durchbruch und Produktverlust*

In allen beschriebenen Versuchen hat das Säulenmaterial nur eine geringe Kapazität für Kontaminanten und eine deutlich höhere Kapazität für das Zielmolekül, dementsprechend tritt zunächst der Durchbruch der Kontaminanten auf und ist als Plateau in der UV Absorption am Ausgang der Säule sichtbar. Erst bei höherem Beladungsvolumen wird die Kapazität für das Zielmolekül erschöpft und der Durchbruch des Zielmoleküls beobachtet. Der Grad des Durchbruchs des Zielmoleküls kann anhand folgender Beziehung berechnet werden:

$$\text{Durchbruch Zielmolekül}\,(\%) = \frac{(UV_X - UV_{Kontaminante})}{(UV_{Gesamt} - UV_{Kontaminante})} \times 100$$

wobei,

$UV_X$ das UV Signal bei einem bestimmten Beladungsvolumen x [mAU],
$UV_{Kontaminante}$ das UV Signal bei kompletten Durchbruch der Kontaminante (Basislinie) [mAU] und
$UV_{Gesamt}$ das UV Signal bei komplettem Durchbruch der Kontaminante und des Zielmoleküls [mAU] sind.

Der relative Produktverlust kann direkt aus der Durchbruchskurve über das Verhältnis der Flächenintegrale der UV Absorption von beladenem Zielmolekül und nicht gebundenem Zielmolekül (unterhalb der Durchbruchskurve) berechnet werden:

$$\text{Produktverlust}\,(\%) = \frac{\text{Fläche unterhalb der Kurve}}{\text{Fläche Gesamt}} \times 100$$

Beispiel 1 - Einfluss der Betthöhe des nachgeschalteten Membranadsorbers

Im ersten Beispiel wird IgG (SeraCare, HS-475) mit Hilfe von Protein A Affinitätschromatographie aufgereinigt. Protein A bindet mit sehr hoher Affinität an die Fc Region der schweren Kette von Immunglobulinen, wobei Kontaminanten nur sehr schwach an das Säulenmaterial binden und beim Beladen der Säule im Durchlauf sind. Als Hauptmedium (entsprechend dem ersten Chromatographiesystem) wird eine Protein A Säule (HiTrap Protein A, Art Nr. 17-0403-01, GE Healthcare) verwendet, die ein Bettvolumen von 5 ml und eine Bindungskapazität von 30 mg IgG/ml aufweist. Durch die Nachschaltung eines Membranadsorbers ("MA 1", Sartobind® Protein A Membran Art. Nr. 93PRAP06HB-12-A, Kapazität 6.5 mg/ml) mit variabler Dimensionierung (0,1-2 ml) wird der Einfluss der Betthöhe auf den Produktverlust, die Nachweisempfindlichkeit und den Rückdruck beim Beladen des Aufbaus untersucht (Tabelle 1). Die Beladungsversuche werden dazu mit einer Mischung aus IgG (2,5 mg/ml) als Zielmolekül und BSA (1 mg/ml) als Kontaminante durchgeführt.

Tab. 1: Anzahl der Lagen, Größe der Gesamtfläche, des Volumens und des Totvolumens der einzelnen Membranadsorber (MA 1)

| Betthöhe, mm | Anzahl der Lagen | Anströmfläche, cm$^2$ | Gesamtflächefläche, cm$^2$ | Membranvolumen, ml | Totvolumen, ml |
|---|---|---|---|---|---|
| 0 | 0 | 0 | 0 | 0 | 0 |
| 0,2 | 1 | 5 | 5 | 0,1 | 0,5 |
| 1 | 5 | 5 | 25 | 0,5 | 0,6 |
| 2 | 10 | 5 | 50 | 1,0 | 1,1 |
| 3 | 15 | 5 | 75 | 1,5 | 1,2 |
| 4 | 20 | 5 | 100 | 2,0 | 1,5 |

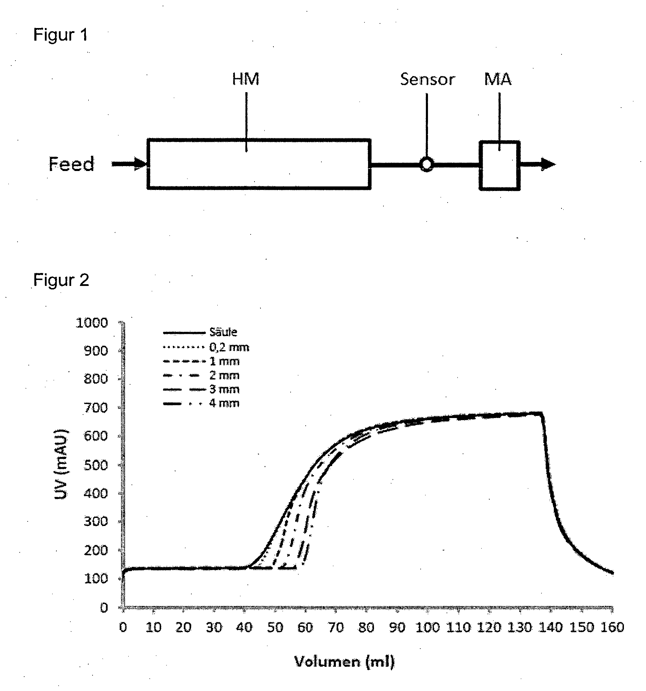

Dazu wird jeder Aufbau zunächst mit 50 ml Puffer A equilibriert, dann mit 140 ml IgG (2,5 mg/ml) und BSA (1 mg/ml) in Puffer A beladen, mit 70 ml Puffer A gewaschen und anschließend mit 50 ml Puffer B eluiert. Im Chromatogramm ist der frühe Durchbruch der Kontaminante (BSA, UVKontaminante = 135 mAU) gefolgt von dem Durchbruch der Zielmoleküle (40-60 ml) ($UV_{Gesamt}$ = 680 mAU) zu sehen (vgl. Figur 2). Der Einfluss der nachgeschalteten Membranadsorber lässt sich dabei in einer Zunahme der Steilheit der Durchbruchskurven mit zunehmender Größe des Adsorbers und einer erhöhten Kapazität (späterer Durchbruch) erkennen (vgl. Figur 2).

Für die praktische Anwendung kann die zusätzliche Kapazität, die durch den Membranadsorber eingebracht wird, zur optimalen Ausnutzung der Säule bei einer Minimierung des Produktverlustes eingesetzt werden. Als Abbruchkriterium für die Beladung dient im Falle der Durchbruchskurve ohne nachgeschalteten Membranadsorber das Maximum der ersten Ableitung der UV Absorption. Im Falle der Experimente mit nachgeschaltetem Membranadsorber wird das Maximum der Differenz des Signals (in mAU) des Sensors vor dem Membranadsorber in einem festgelegten Volumenintervall als Abbruchkriterium ($\Delta UV = UV_{X+Y} - UV_X$) genutzt. Das Volumenintervall kann basierend auf der Konzentration des Zielmoleküls in der Feedlösung, der Kapazität des Membranadsorbers und der Empfindlichkeit des verwendeten Sensors gewählt werden. In diesem und den nachfolgenden Beispielen wird das Volumenintervall konservativ, ohne Berücksichtigung der Kapazität des nachgeschalteten Membranadsorbers, mit dem Totvolumen des nachgeschalteten Membranadsorbers angesetzt ($\Delta UV = UV_{X+TV} - UV_X$).

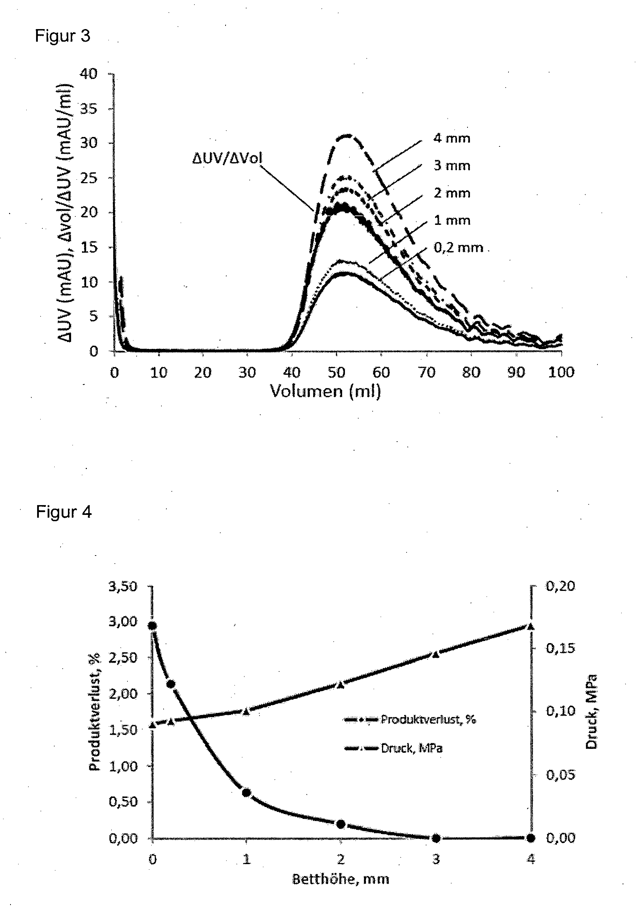

Es lässt sich ein deutlicher Zusammenhang aus der Größe der gewählten Volumendifferenz und der Amplitude des $\Delta UV$ Signals erkennen, die Position der Maximalwerte auf der x-Achse bleibt jedoch gleich (vgl. Figur 3). Auffällig ist die größere Amplitude des $\Delta UV$ Signals im Vergleich zur ersten Ableitung bei größeren Membranvolumina (1,5 ml und 2 ml), was bei schlecht detektierbaren Signalen (bei hoher Konzentration von Kontaminanten und niedriger Konzentration von Zielmolekülen) einen Vorteil bei Bestimmung des Durchbruchs darstellt. Unter Verwendung der beschriebenen Abbruchkriterien wird deutlich, dass durch die Vergrößerung des Volumens des nachgeschalteten Membranadsorbers eine Minimierung des Produktverlusts von 3 % auf 0 % erzielt werden kann (vgl. Tab. 2).

**Tab. 2:** $\Delta UV$ Signal, Druck während der Beladung, Durchbruch der Zielsubstanz (IgG) in %, und entsprechender Produktverlust

| Betthöhe, mm | $\Delta UV$ (mAU) oder (mAU/ml) | Durchschnittlicher Druck während der Beladung, MPa | Durchbruch, % | Produktverlust, % |
|---|---|---|---|---|
| 0 | 20,7 | 0,09 | 32,4 | 2,95 |
| 0,2 | 11,3 | 0,09 | 29,9 | 2,14 |
| 1 | 13,2 | 0,10 | 14,8 | 0,63 |
| 2 | 23,4 | 0,12 | 0,6 | 0,20 |
| 3 | 25,1 | 0,15 | 0 | 0,00 |
| 4 | 31,1 | 0,17 | 0 | 0,00 |

Gleichzeitig ist aber auch eine Zunahme des Rückdrucks beobachtbar, die mit der Größe des nachgeschalteten Membranadsorbers korreliert (vgl. Tab. 2 und Figur 4). Mit der zunehmenden Größe des Membranadsorbers ergibt sich somit ein Gegensatz aus dem Produktverlust (der gegen null geht) und der Zunahme des Rückdrucks bei der Beladung. Dieser Zusammenhang kann für die Dimensionierung des Membranadsorbers entscheidend sein, da dieser idealerweise in bereits bestehende Systeme mit einem maximal zulässigen Rückdruck integrierbar ist.

Bis zum vollständigen Durchbruch des Zielmoleküls konnte die hier verwendete 5 ml Protein A Säule mit 152 mg IgG beladen werden. In der Regel wird diese Kapazität jedoch nicht vollständig ausgenutzt. Um einem Verlust des Zielmoleküls vorzubeugen, werden Säulenmaterialien im Prozessmaßstab nur zu ca. 60 % ihrer Kapazität (im Falle der hier verwendeten Säule entspräche dies 91 mg IgG) beladen. In dem hier beschriebenen Aufbau kann bis zum Erreichen des Abbruchkriteriums eine Beladung der Säule mit 127 mg IgG realisiert werden, was einer Ausnutzung der vorhandenen Bindungskapazität der Säule von 83 % entspricht.

Insgesamt lässt sich festhalten, dass der nachgeschaltete Membranadsorber Zielmoleküle, die bei einer zuverlässigen Detektion des Durchbruchs nach der Säule verloren gegangen sind, auffangen kann. Dabei kommt dem Aufbau die Eigenschaft der steileren Durchbruchskurve nach dem Membranadsorber zugute, da dadurch bereits bei relativ kleiner Dimensionierung des Membranadsorbers (6.5 % der Kapazität des Hauptmediums, 3 mm Betthöhe) der Verlust vollständig vermieden werden kann.

Referenzbeispiel 2 - Größe der Anströmfläche des nachgeschalteten Membranadsorbers

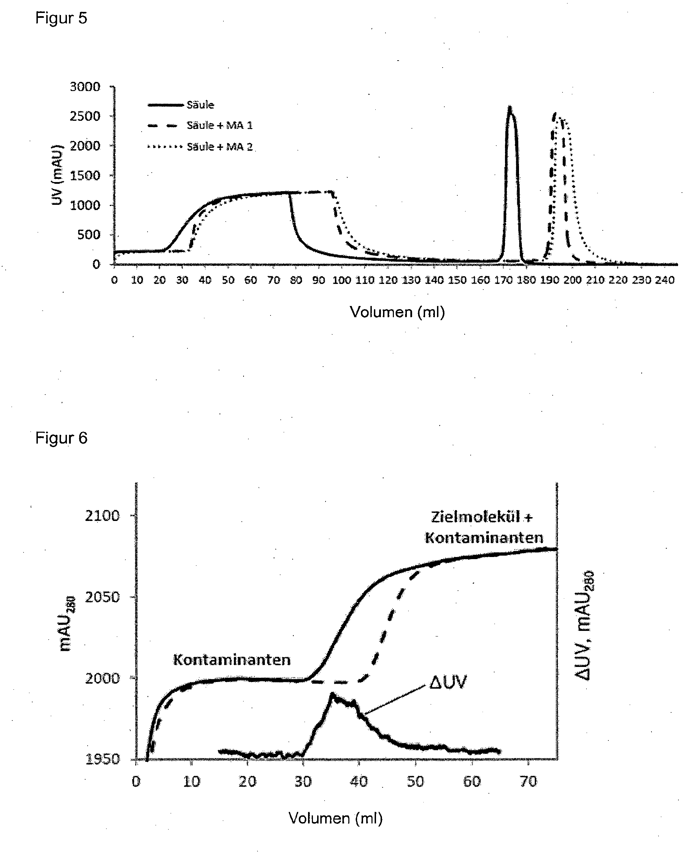

Ein wichtiger Parameter bei der Chromatographie ist die Elution der Zielmoleküle. Hierbei ist darauf zu achten, dass das Elutionsvolumen durch den Einbau eines zusätzlichen Membranadsorbers nicht übermäßig gesteigert werden sollte. Des Weiteren ist die Peaksymmetrie bei der Elution ein wichtiger Parameter. Die Geometrie des Membranadsorbers hat entscheidenden Einfluss auf die lineare Flussgeschwindigkeit der Probe durch die Membran und kann somit Einfluss auf die Bindung und die Elution der Zielmoleküle haben. Zur Untersuchung dieses Effekts wird in diesem Experiment eine Lösung aus IgG (5 mg/ml) und BSA (1 mg/ml) auf eine Protein A Säule beladen und anschließend eluiert (vgl. Figur 5). In einem Fall wird die Säule (5 ml) allein, in dem anderen Fall mit einem nachgeschalteten Membranadsorber mit 5 cm$^2$ Anströmfläche ("MA1") und in dem weiteren Fall mit einem nachgeschalteten Membranadsorber mit 20 cm$^2$ Anströmfläche ("MA2"), mit in beiden Fällen gleichem Bettvolumen von 2 ml verwendet, um so eine Variation der Anströmfläche um den Faktor 4 zu erreichen (vgl. Tab. 3).

**Tab. 3**: Eigenschaften der verwendeten Membranadsorber

|  | MA 1 | MA 2 |
|---|---|---|
| Fluss | Axial | Axial |
| Anzahl der Lagen | 20 | 5 |
| Betthöhe | 4 mm | 1 mm |
| Bettvolumen | 2 ml | 2 ml |
| Anströmfläche | 5 cm$^2$ | 20 cm$^2$ |
| Durchmesser | 30 mm | 57 mm |
| Membranfläche | 100 cm$^2$ | 100 cm$^2$ |
| Totvolumen | 1,5 ml | 2,8 ml |

Zur Überprüfung der Effekte der geänderten Anströmflache wurden die Einheiten nun mit 50 ml Puffer A (siehe Material und Methoden) equilibriert, anschließend mit 80 ml (nur Säule) oder 100 ml (Säule + Membranadsorber) IgG (5 mg/ml) und BSA (1 mg/ml) in Puffer A beladen, mit 70 ml Puffer A gewaschen, und danach mit 60 ml Puffer B eluiert (vgl. Figur 5).

Neben dem bereits beschriebenen, steileren Anstieg der Durchbruchskurve des Zielmoleküls in Gegenwart der Membranadsorber (vgl. Figur 5, sowie Beispiel 1), ist auch eine Veränderung der Form des Elutionspeaks beobachtbar. Insbesondere der Tailing-Faktor (T) des Elutionspeaks ist ein wichtiger Parameter zur Beschreibung dieses Umstands und lässt sich mit einer einfachen Gleichung darstellen:

$$T = \frac{a+b}{2a}.$$

Dabei sind a und b die Distanzen, die die Ausdehnung des Elutionspeaks (Fronting und Tailing) vom Peakmittelpunkt bei einem 1/10 der maximalen Peakhöhe (h) beschreiben (Figur 5). Bei einem Wert von $T \neq 1$ ist der Peak asymmetrisch, wobei ein Wert für T < 1 Fronting und ein Wert von T > 1 Tailing beschreibt. In allen Fällen ist ein Tailing des Elutionspeaks beobachtbar, wobei im Fall ohne Membranadsorber das geringste Tailing, im Fall von MA 1 nur ein geringer Anstieg des Tailings und im Fall von MA 2 ein deutliches Tailing zu beobachten ist (vgl. Figur 5 und Tab. 4).

Des Weiteren wurde das Elutionsvolumen gemessen, das benötigt wird, um das Zielmolekül von der Säule zu eluieren (vgl. Tab. 4). Die Ergebnisse zeigen generell einen Anstieg des Elutionsvolumens in der Gegenwart eines Membranadsorbers, wobei der Membranadsorber mit der größeren Anströmfläche (MA 2) eine deutliche Peakverbreiterung und somit eine deutlichere Erhöhung des Elutionsvolumens (Faktor 2,2) im Vergleich zu dem Membranadsorber mit der kleineren Anströmfläche (MA 1, Faktor 1,2) zeigt.

**Tab. 4**: Einfluss der unterschiedlichen Anströmflächen auf die Elution von Zielmolekülen

|  | Säule | Säule + MA 1 | Säule + MA 2 |
|---|---|---|---|
| Tailing-Faktor (T) | 1,31 | 1,39 | 1,90 |
| Elutionsvolumen (ml) | 7,6 | 9,5 | 16,8 |
| Volumenanstieg | 1,0 | 1,2 | 2,2 |

Es ist davon auszugehen, dass sowohl das Totvolumen des Membranadsorbers als auch die lineare Flussgeschwindigkeit entlang der radialen Achse Einfluss auf die Bindung der Zielmoleküle auf der Membran haben. Somit lässt sich die Erhöhung des Elutionsvolumens bei der Einheit mit breiter Anströmfläche auf Unterschiede in den Verweilzeiten in radialer Flussrichtung erklären. Dies kann für Anwendungen, bei denen es auf eine Minimierung des Elutionsvolumens ankommt, entscheidend sein (dies ist generell bei vielen Anwendungen der Fall, da es oft darum geht, Kosten für Puffer und weitere Verdünnungen z.B. zur Einstellung des Puffers vor dem Einbringen in einen weiteren Prozessschritt zu sparen) und ist grundsätzlich bei der Anwendung der Methode zu beachten.

Beispiel 3 - Anwendbarkeit der Methode auf Ionentauscherchromatographie

Um die generelle Anwendbarkeit der Methode auf andere chromatographische Matrizes zu zeigen, wird im folgenden Beispiel eine Mischung aus Lysozym (5 mg/ml) und Bovinem Serum Albumin (BSA, 2 mg/ml) mittels Anionentauscherchromatographie bei einem pH-Wert von 7,4 getrennt. Während Lysozym einen isoelektrischen Punkt von 11,4 hat, besitzt BSA einen isoelektrischen Punkt von 4,7. Somit hat Lysozym bei dem verwendeten pH-Wert eine positive Nettoladung und bindet nur in sehr geringem Maße an den Anionentauscher, während BSA negativ geladen ist und somit eine starke Affinität für die positiv geladenen quarternären Ammoniumgruppen $R-CH_2-N^+(CH_3)_3$ des Anionentauschers aufweist. Lysozym dient somit als Modell für eine Kontaminante und BSA als Modell für das Zielmolekül. Sowohl bei dem Hauptmedium (Sartobind® Q 3 ml Membranadsorber) als auch bei dem kleiner dimensionierten Membranadsorber (MA1, Sartobind® Q 0,4 ml) sind die Liganden als auch die Membran identisch, beide weisen eine dynamische Bindungskapaziät von 30 mg BSA/ml auf (siehe Tab. 5). Die beiden Medien unterscheiden sich jedoch in der Dimensionierung, der zweite Membranadsorber ist kleiner und besitzt nur 13 % der Kapazität des Hauptmediums.

**Tab. 5:** Parameter des Aufbaus zur Trennung von BSA und Lysozym auf Anionentauscher Membranen (MV = Membranvolumen)

| Parameter | Hauptmedium | MA 1 |
| --- | --- | --- |
| Fluss | Radial | Axial |
| Bindungskapazität (BSA) | 30 mg/ml MV | 30 mg/ml MV |
| Bettvolumen | 3 ml | 0,4 ml |
| Totvolumen | 4,3 ml | 0,9 ml |

Die Detektion des Beladungszustands erfolgt am Ausgang des Hauptmediums. Hierzu wird wie bereits beschrieben die Steigung des UV Signals durch den ΔUV Wert ermittelt. Das Volumenintervall entspricht in diesem Beispiel wiederum dem Totvolumen des nachgeschalteten Membranadsorbers (MA1, 0,9 ml, vgl. Beispiel 1). Als Abbruchkriterium für die Beladung wird der Maximalwert der ΔUV Kurve gewählt (ca. 36 ml, vgl. Figur 6).
Bei diesem Verfahren wird ohne nachgeschalteten Membranadsorber ein Produktverlust von 2 % erreicht (vgl. Tab. 6).

**Tab. 6:** Detektion des Durchbruchs von BSA

| | Beladungsvolumen, ml | mAU (280 nm) | Durchbruch, % | Produktverlust, % |
| --- | --- | --- | --- | --- |
| Hauptmedium | 36 | 2024 | 29 | 2 |
| Hauptmedium + MA 1 | 36 | 1997 | 0 | 0 |

Durch den nachgeschalteten Membranadsorber wird jedoch ein Großteil der Zielmoleküle aufgefangen, die nicht an das Hauptmedium gebunden haben (Figur 6 gestrichelte Linie). So kann bei der Verwendung des Membranadsorbers bei Beladung des Hauptmediums bis zum Durchbruch des Zielmoleküls und somit optimaler Ausnutzung der Kapazität, der Produktverlust minimiert werden (nicht detektierbar). Das Konzept ist also grundsätzlich nicht auf Protein A Chromatographie beschränkt und leicht auf andere Systeme übertragbar.

**Patentansprüche**

1. Vorrichtung zum Trennen und/oder Isolieren von proteinhaltigen Substanzen in bzw. aus einem Gemisch in einem Fluid, bestehend aus einem ersten Chromatographiesystem, welches eine oder mehrere Chromatographiematrizes umfasst, einem einzigen, dem ersten Chromatographiesystem nachgeschalteten Sensor zur Detektion der im Fluid enthaltenen Substanzen und einer dem Sensor nachgeschalteten zweiten Chromatographiematrix, wobei die zweite Chromatographiematrix dem ersten Chromatographiesystem so nachgeschaltet ist, dass das das erste Chromatographiesystem verlassende Fluid durch die zweite Chromatographiematrix leitbar ist, wobei die Kapazität der zweiten Chromatographiematrix kleiner als die Kapazität des ersten Chromatographiesystems ist und wobei die erste Chromatographiematrix eine konvektive Chromatographiematrix wie ein Membranadsorber oder ein Monolith ist.

2. Vorrichtung nach Anspruch 1, wobei das Messprinzip des Sensors auf UV oder IR Absorptionsspektroskopie, Terahertzspektroskopie, Manometrie, Konduktometrie, Potentiometrie, Refraktometrie, Radiometrie, Fluoreszenzspektroskopie insbesondere des Oberflächenfluoreszenzquenchings, ATR (Abgeschwächte Total Reflektion)-Infrarotspektroskopie insbesondere der Oberflächenplasmonenresonanzspektroskopie (SPRS), Potentiometrie, Polarimetrie, Impendanzspektroskopie, NMR-Spektroskopie, Raman-Spektroskopie, Turbidimetrie, Nephelometrie und der Ultraschall Laufzeit basiert, oder eine Kombination von mehreren Techniken als Detektionsprinzip für den Sensor umfasst.

3. Vorrichtung nach einem der Ansprüche 1 oder 2, wobei das erste Chromatographiesystem einen Liganden und die zweite Chromatographiematrix einen Liganden aufweisen, wobei der Ligand des ersten Chromatographiesystems und der Ligand der zweiten Chromatographiematrix jeweils über eine gleiche chemische und/oder physikalische Wechselwirkung mit mindestens einer Substanz in dem Gemisch interagieren, ausgewählt aus der Gruppe der Ionenaustauscher, salztoleranten Liganden, Chelatbildner, thiophilen oder hydrophoben Liganden verschiedener Kettenlängen und Konfigurationen, "Reversed-Phase"-Liganden, reaktiven und anderen Farbstoffen, der anorganischen Moleküle und Ionen, deren organischen und anorganischen Verbindungen, Affinitätsliganden, hochmolekularen Liganden, Enzyme und Untereinheiten sowie Teilen davon, Strukturproteine, Rezeptoren und Effektoren sowie Teilen davon, Viren und Teilen davon, Xenobiotika, Pharmazeutika und pharmazeutischen Wirkstoffe, Alkaloide, Antibiotika, Biomimetika und der Katalysatoren.

4. Vorrichtung nach einem der Ansprüche 1 bis 3, wobei das erste Chromatographiesystem und die zweite Chromatographiematrix mindestens einen gleichen Liganden aufweisen, der über mindestens eine chemische und/oder physikalische Wechselwirkung mit mindestens einer Substanz in dem Gemisch interagiert, ausgewählt aus der Gruppe der Ionenaustauscher, salztoleranten Liganden, Chelatbildner, thiophilen oder hydrophoben Liganden verschiedener Kettenlängen und Konfigurationen, "Reversed-Phase"-Liganden, reaktiven und anderen Farbstoffen, der anorganischen Moleküle und Ionen, deren organischen und anorganischen Verbindungen, Affinitätsliganden, hochmolekularen Liganden, Enzyme und Untereinheiten sowie Teilen davon, Strukturproteine, Rezeptoren und Effektoren sowie Teilen davon, Viren und Teilen davon, Xenobiotika, Pharmazeutika und pharmazeutischen Wirkstoffe, Alkaloide, Antibiotika, Biomimetika und der Katalysatoren.

5. Vorrichtung nach einem der Ansprüche 1 bis4, wobei das erste Chromatographiesystem und die zweite Chromatographiematrix jeweils mindestens einen unterschiedlichen Liganden aufweisen, der über mindestens eine chemische und/oder physikalische Wechselwirkung mit mindestens einer Substanz in dem Gemisch interagiert, ausgewählt aus der Gruppe der Ionenaustauscher, salztoleranten Liganden, Chelatbildner, thiophilen oder hydrophoben Liganden verschiedener Kettenlängen und Konfigurationen, "Reversed-Phase"-Liganden, reaktiven und anderen Farbstoffen, der anorganischen Moleküle und Ionen, deren organischen und anorganischen Verbindungen, Affinitätsliganden, hochmolekularen Liganden, Enzyme und Untereinheiten sowie Teilen davon, Strukturproteine, Rezeptoren und Effektoren sowie Teilen davon, Viren und Teilen davon, Xenobiotika, Pharmazeutika und pharmazeutischen Wirkstoffe, Alkaloide, Antibiotika, Biomimetika und der Katalysatoren.

6. Vorrichtung nach einem der Ansprüche 1 bis 5, wobei das das erste Chromatographiesystem verlassende Fluid vollständig durch die zweite Chromatographiematrix leitbar ist.

7. Vorrichtung nach einem der Ansprüche 1 bis 6, wobei der Sensor in Bezug auf das Zielmolekül nicht destruktiv ist.

8. Vorrichtung nach einem der Ansprüche 1 bis 7, wobei der Sensor ein UV, IR, pH, Druck- oder Leitfähigkeits-Sensor ist.

9. Verfahren zum Trennen und/oder Isolieren von proteinhaltigen Substanzen in bzw. aus einem Gemisch in einem Fluid, umfassend die Schritte des Hindurchleitens des Fluids durch die Vorrichtung nach einem der Ansprüche 1 bis 8 und des Sammelns des hindurchgeleiteten Fluids.

10. Verfahren nach Anspruch 9, wobei das Gemisch eine Lösung eines synthetisch oder biologisch hergestellten Produktes, oder ein Naturstoff ist.

**11.** Verfahren nach Anspruch 9, wobei das Gemisch eine antikörperhaltige Lösung ist.

**12.** Verwendung der Vorrichtung nach einem der Ansprüche 1 bis 8 zum Trennen und/oder Isolieren eines Proteinsin bzw. aus einem Gemisch in einem Fluid.

**Claims**

**1.** A device for separating and/or isolating protein content substances in or from a mixture in a fluid consisting of a first chromatography system, which comprises one or a plurality of chromatography matrices, a single sensor arranged downstream of the first chromatography system for detecting the substances contained in the fluid and a second chromatography matrix arranged downstream of the sensor, wherein the second chromatography matrix is arranged downstream such that the fluid exiting the first chromatography system is conductible through the second chromatography matrix, wherein the capacity of the second chromatography matrix is less than the capacity of the first chromatography system and wherein the second chromatography matrix is a convective chromatography matrix such as a membrane adsorber or a monolith.

**2.** The device according to claim 1, wherein the measuring principle of the sensor is based on UV or IR absorption spectroscopy, terahertz spectroscopy, manometry, conductometry, potentiometry, refractometry, radiometry, fluorescence spectroscopy in particular of the surface fluorescence quenching, ATR (weakened total reflection) infrared spectroscopy in particular of the surface plasmon resonance spectroscopy (SPRS), potentiometry, polarimetry, impedance spectroscopy, NMR spectroscopy, Raman spectroscopy, turbidimetry, nephelometry and ultrasound run time, or comprises a combination of several techniques as a detection principle for the sensor.

**3.** The device according to claim 1 or 2, wherein the first chromatography system has a ligand and the second chromatography matrix has a ligand, wherein the ligand of the first chromatography system and the ligand of the second chromatography matrix each interact with at least one substance in the mixture via an identical chemical and/or physical interaction, selected from the group of ion exchangers, salt-tolerant ligands, chelating agents, thiophilic or hydrophobic ligands of various chain lengths and configurations, "Reversed-Phase" ligands, reactive and other dye substances, of inorganic molecules and ions, their organic and inorganic compounds, affinity ligands, high molecular ligands, enzymes and subunits as well as parts thereof, structure proteins, receptors and effectors as well as parts thereof, viruses and parts thereof, xenobiotics, pharmaceuticals and pharmaceutical agents, alkaloids, antibiotics, biomimetics and their catalysts.

**4.** The device according to any of claims 1 to 3, wherein the first chromatography system and the second chromatography matrix have at least one same ligand which interacts with at least one substance in the mixture via at least a chemical and/or physical interaction, selected from the group of ion exchangers, salt-tolerant ligands, chelating agents, thiophilic or hydrophobic ligands of various chain lengths and configurations, "Reversed-Phase" ligands, reactive and other dye substances, of inorganic molecules and ions, their organic and inorganic compounds, affinity ligands, high molecular ligands, enzymes and subunits as well as parts thereof, structure proteins, receptors and effectors as well as parts thereof, viruses and parts thereof, xenobiotics, pharmaceuticals and pharmaceutical agents, alkaloids, antibiotics, biomimetics and their catalysts.

**5.** The device according to any of claims 1 to 4, wherein the first chromatography system and the second chromatography matrix have at least one different ligand which interacts with at least one substance in the mixture via at least a chemical and/or physical interaction, selected from the group of ion exchangers, salt-tolerant ligands, chelating agents, thiophilic or hydrophobic ligands of various chain lengths and configurations, "Reversed-Phase" ligands, reactive and other dye substances, of inorganic molecules and ions, their organic and inorganic compounds, affinity ligands, high molecular ligands, enzymes and subunits as well as parts thereof, structure proteins, receptors and effectors as well as parts thereof, viruses and parts thereof, xenobiotics, pharmaceuticals and pharmaceutical agents, alkaloids, antibiotics, biomimetics and their catalysts.

**6.** The device according to any of claims 1 to 5, wherein the fluid exiting the first chromatography system is completely conductible through the second chromatography matrix.

**7.** The device according to any of claims 1 to 6, wherein the sensor is not destructive with respect to the target molecule.

**8.** The device according to any of claims 1 to 7, wherein the sensor is a UV, IR pH, pressure or conductivity sensor.

**EP 3 167 283 B1**

9. A method for separating and/or isolating protein content substances in or from a mixture in a fluid, comprising the steps of conducting the fluid through the device according to any of claims 1 to 8 and collecting the conducted fluid.

10. The method according to claim 9, wherein the mixture is a solution of a synthetic or biologically produced product, or is a natural substance.

11. The method according to claim 9, wherein the mixture is an antibody containing solution.

12. Use of the device according to any of claims 1 to 8 for separating and/or isolating a protein in or from a mixture in a fluid.

**Revendications**

1. Dispositif destiné à séparer et/ou isoler des substances protéiniques dans ou hors d'un mélange dans un fluide, constitué d'un premier système de chromatographie, lequel comprend une ou plusieurs matrices de chromatographie, d'un unique capteur monté en aval du premier système de chromatographie pour détecter les substances contenues dans le fluide et d'une seconde matrice de chromatographie montée en aval du capteur, dans lequel la seconde matrice de chromatographie est montée en aval du premier système de chromatographie de telle sorte que le fluide qui quitte le premier système de chromatographie puisse être guidé à travers la seconde matrice de chromatographie, dans lequel la capacité de la seconde matrice de chromatographie est inférieure à la capacité du premier système de chromatographie, et dans lequel la seconde matrice de chromatographie est une matrice de chromatographie par convection tel un adsorbeur à membrane ou un monolithe.

2. Dispositif selon la revendication 1, dans lequel le principe de mesure du capteur se base sur une spectroscopie d'absorption UV ou IR, une spectroscopie térahertz, une manométrie, une conductométrie, une potentiométrie, une réfractométrie, une radiométrie, une spectroscopie de fluorescence en particulier de l'extinction de fluorescence de surface, une spectroscopie infrarouge ATR (réflectance totale atténuée) en particulier la spectroscopie à résonance des plasmons de surface (SPRS), une potentiométrie, une polarimétrie, une spectroscopie d'impédance, une spectroscopie RMN, une spectroscopie Raman, une turbidimétrie, une néphélométrie et le temps de transit ultrasonique, ou comprend une combinaison de plusieurs techniques en tant que principe de détection pour le capteur.

3. Dispositif selon la revendication 1 ou 2, dans lequel le premier système de chromatographie présente un ligand et la seconde matrice de chromatographie présente un ligand, dans lequel le ligand du premier système de chromatographie et le ligand de la seconde matrice de chromatographie interagit respectivement via une même interaction chimique et/ou physique avec au moins une substance dans le mélange, sélectionnée dans le groupe des échangeurs d'ions, des ligands tolérants au sel, des agents chélateurs, des ligands thiophiles ou hydrophobes de longueurs de chaîne et configurations différentes, des ligands « en phase inversée », des colorants réactifs et autres, des molécules et ions inorganiques, de leurs composés organiques et inorganiques, des ligands d'affinité, des ligands à haut poids moléculaire, des enzymes et sous-unités ainsi que parties de celles-ci, des protéines structurales, des récepteurs et effecteurs ainsi que parties de ceux-ci, des virus et parties de ceux-ci, des xénobiotiques, des produits pharmaceutiques et agents pharmaceutiques, des alcaloïdes, des antibiotiques, des produits biomimétiques, et des catalyseurs.

4. Dispositif selon l'une des revendications 1 à 3, dans lequel le premier système de chromatographie et la seconde matrice de chromatographie présentent au moins un même ligand qui interagit via au moins une interaction chimique et/ou physique avec au moins une substance dans le mélange, sélectionnée dans le groupe des échangeurs d'ions, des ligands tolérants au sel, des agents chélateurs, des ligands thiophiles ou hydrophobes de longueurs de chaîne et configurations différentes, des ligands « en phase inversée », des colorants réactifs et autres, des molécules et ions inorganiques, de leurs composés organiques et inorganiques, des ligands d'affinité, des ligands à haut poids moléculaire, des enzymes et sous-unités ainsi que parties de celles-ci, des protéines structurales, des récepteurs et effecteurs ainsi que parties de ceux-ci, des virus et parties de ceux-ci, des xénobiotiques, des produits pharmaceutiques et agents pharmaceutiques, des alcaloïdes, des antibiotiques, des produits biomimétiques, et des catalyseurs.

5. Dispositif selon l'une des revendications 1 à 4, dans lequel le premier système de chromatographie et la seconde matrice de chromatographie présentent respectivement au moins un ligand différent qui interagit via au moins une interaction chimique et/ou physique avec au moins une substance dans le mélange, sélectionnée dans le groupe des échangeurs d'ions, des ligands tolérants au sel, des agents chélateurs, des ligands thiophiles ou hydrophobes de longueurs de chaîne et configurations différentes, des ligands « en phase inversée », des colorants réactifs et autres, des molécules et ions inorganiques, de leurs composés organiques et inorganiques, des ligands d'affinité, des ligands à haut poids moléculaire, des enzymes et sous-unités ainsi que parties de celles-ci, des protéines structurales, des récepteurs et effecteurs ainsi que parties de ceux-ci, des virus et parties de ceux-ci, des xénobiotiques, des produits pharmaceutiques et agents pharmaceutiques, des alcaloïdes, des antibiotiques, des produits biomimétiques, et des catalyseurs.

6. Dispositif selon l'une des revendications 1 à 5, dans lequel le fluide qui quitte le premier système de chromatographie peut être entièrement guidé à travers la seconde matrice de chromatographie.

7. Dispositif selon l'une des revendications 1 à 6, dans lequel le capteur n'est pas destructeur en ce qui concerne la molécule cible.

8. Dispositif selon l'une des revendications 1 à 7, dans lequel le capteur est un capteur d'UV, d'IR, de pH, de pression ou de conductivité.

9. Procédé destiné à séparer et/ou isoler des substances protéiniques dans ou hors d'un mélange dans un fluide, qui comprend les étapes qui consistent à guider le fluide à travers le dispositif selon l'une des revendications 1 à 8 et à collecter le fluide guidé au travers.

10. Procédé selon la revendication 9, dans lequel le mélange est une solution d'un produit fabriqué par synthèse ou biologiquement, ou une substance naturelle.

11. Procédé selon la revendication 9, dans lequel le mélange est une solution qui contient un anticorps.

12. Utilisation du dispositif selon l'une des revendications 1 à 8 destiné à séparer et/ou isoler une protéine dans ou hors d'un mélange dans un fluide.

Figur 1

Figur 2

Figur 3

Figur 4

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010083859 A1
- WO 2010151214 A1
- WO 9934220 A2
- US 7901581 B2
- EP 1718668 B1